Europäisches Patentamt

European Patent Office

Office européen des brevets

(11) Publication number: **0 273 778**
**A2**

(12) # EUROPEAN PATENT APPLICATION

(21) Application number: 87311552.1

(22) Date of filing: 31.12.87

(51) Int. Cl.⁴: **A 61 K 37/00**
**A 61 K 39/00**

(30) Priority: 31.12.86 US 948159    04.12.87 US 126221

(43) Date of publication of application:
06.07.88  Bulletin  88/27

(84) Designated Contracting States:
AT BE CH DE ES FR GB GR IT LI LU NL SE

(71) Applicant: **CETUS CORPORATION**
**1400 Fifty-Third Street**
**Emeryville California 94608  (US)**

(72) Inventor: **Warren, Mary Kim**
**486 Joaquin Avenue**
**San Leandro California 94577  (US)**

**Ralph, Peter**
**119 Crest View Drive**
**Orinda California 94563  (US)**

(74) Representative: **Bizley, Richard Edward et al**
**BOULT, WADE & TENNANT 27 Furnival Street**
**London EC4A 1PQ  (GB)**

(54) Synergistic behavior of csf-1 and G-csf.

(57) Two colony stimulating factors, G-CSF and CSF-1, are synergistic in their effect on colony formation and stimulation of the immune system.

EP 0 273 778 A2

## Description

## SYNERGISTIC BEHAVIOR OF CSF-1 AND G-CSF

Technical Field

The present invention relates to the use of combinations of lymphokines, produced if desired by recombinant technology, to effect immune system stimulation. More specifically, the invention relates to the synergistic effect of colony stimulating factor-1 (CSF-1) and granulocyte colony stimulating factor (GCSF).

Background Art

The ability of certain factors produced in very low concentration in a variety of tissues to stimulate the growth and development of bone marrow progenitor cells into granulocytes and/or macrophages has been known for nearly 15 years. The presence of such factors in sera, urine samples, and tissue extracts from a number of species is demonstrable using an in vitro assay which measures the stimulation of colony formation by bone marrow cells plated in semisolid culture medium. There are no standard, quantitative in vivo assays. Because these factors induce the formation of such colonies, the factors collectively have been called Colony Stimulating Factors (CSF). A recent review is that of Metcalf, D.,Blood (1986) 67:257.

It has been shown that there are at least four subclasses of human CSF proteins which can be defined according to the types of cells found in the resultant colonies. One subclass, CSF-1, results in almost 100% colonies containing only macrophages. Another subclass, G-CSF, stimulates colonies wherein the majority contain only neutrophilic granulocytes. Other subclasses (GM-CSF) produce both colonies which contain granulocytes and colonies which contain macrophages; and both colonies which contain neutrophilic and eosinophilic granulocytes, and colonies which contain macrophages. In addition, a murine factor called IL-3 induces colonies from murine bone marrow cells which contain all these cell types plus megakaryocytes, erythrocytes, and mast cells, in various combinations. Human IL-3 is also known (Yang, Y.-C., et al, Cell (1986) 47:3-10). These CSFs have been reviewed by Dexter, T. M., Nature (1984) 309:746; Vadas, M. A., et al, J Immunol (1983) 130:793; Metcalf, D., (1985) 229:16-22; and Clark, S.C. and Kamen, R., Science (1987) 236:1229-1237.

Purification methods for various CSF proteins have been published. With respect to CSF-1, Stanley, E.R., et al, J Biol Chem (1977) 252:4305 reported purification of this CSF protein from murine L929 cells to a specific activity of about 1 x 10⁸ units/mg, which also stimulated mainly macrophage production. Waheed, A., et al, Blood (1982) 60:238, described the purification of mouse L-cell CSF-1 to apparent homogeneity using a rabbit antibody column and reported the first 25 amino acids of the murine sequence (BenAvram, C.M., et al, Proc Natl Acad Sci (USA) (1985) 882:4486. Stanley, E.R., et al, J Biol Chem (1977) 252:4305-4312 disclosed a purification procedure for CSFl from human urine and Das, S.K., et al, Blood (1981) 58:630; J Biol Chem (1982) 257:13679 obtained a human urinary CSF-1 at a specific activity of 5 x 10⁷ units/mg which produced only macrophage colonies, and outlined the relationship of glycosylation of the CSF-1 proteins prepared from cultured mouse L-cells and from human urine to their activities. Wang, F.F., et al, J Cell Biochem (1983) 21:263, isolated human urinary CSF-1 to specific activity of 10⁸ U/mg. Waheed, A., et al, disclosed purification of human urinary CSF-1 to a specific activity of 0.7-2.3 x 10⁷ U/mg on a rabbit antibody column (Exp Hemat (1984) 12:434).

Partially purified preparations of various CSFs have also been reported from human and mouse lung-cell conditioned media (Fojo, S.S., et al, Biochemistry (1978) 17:3109; Burgess, A.W., et al, J Biol Chem (1977) 252:1998); from human T-lymphoblast cells (Lusis, A.J., et al, Blood (1981) 57:13; U.S. Patent, 4,438,032); from human placental conditioned medium to apparent homogeneity and specific activity of 7 x 10⁷ U/mg (Wu, M., et al, Biochemistry (1980) 19:3846).

Wu, M., et al, J Biol Chem (1979) 254:6226 reported the preparation of a CSF protein from cultured human pancreatic carcinoma (MIAPaCa) cells which resulted in the growth of murine granulocytic and macrophagic colonies. The resulting protein had a specific activity of approximately 7 x 10⁷ units/mg. In addition, these cells produce G-CSF as shown by the ability of this factor to induce formation of granulocyte colonies in seven day bone marrow cultures (Wu, M C J Clin Invest (1981) 67:1588) Many of these proteins are now available in recombinant orm.

For example, cDNA encoding human CSF-1, has been cloned and expressed (Kawasaki, E.S., et al, Science (1985) 230:292-296). Recovery of a clone encoding a "long form" of human CSF-1 is reported by Wong, G.G., et al, Science (1987) 235:1504-1509 and by Ladner, M.D., et al, EMBO J (1987) 6:2693-2698.

The cloning and expression of cDNA encoding human G-CSF has been reported by Nagata, S., et al (Nature (1986) 319:415 and EMBO J (1986) 5:575) and by Devlin, J.J., et al, (J Leuk Biol (1987) 41:302-306). What appears to be a G-CSF, has been purified (EPO publication 0169,566), cloned and, perhaps, expressed by Souza, et al, Science (1986) 232:61-65.

Other, different, CSF proteins are also known. Murine and human GM-CSF have been shown to be distinct from other CSFs, e.g., CSF-1, by Gough, et al, Nature (1984) 309:763-767. Murine IL-3 has been cloned by Fung, M. C., et al, Nature (1984) 307:233. Human and gibbon IL-3 have been cloned by Yang, Y.-C., et al, Cell (1986) supra and by Dorssers, L., et al, Gene (1987), in press. See also Yokota, T., et al, Proc Natl Acad Sci USA (1984) 81:1070-1074; Wong, G.G., et al, Science (1985) 228:810-815; Lee, F., et al, Proc Natl Acad Sci USA (1985) 82:4360-4364; and Cantrell, M.A., et al, Proc Natl Acad Sci USA (1985) 82:6250-6254.

## Disclosure of the Invention

The invention provides a means to augment the effect of either CSF-1 and G-CSF by combining each with the other in therapeutic use. Surprisingly, use of these two factors is synergistic with respect to eliciting enhancement of the immune system. Together their effect is greater than the sum obtained from simple addition of the effect of either acting alone. It is therefore possible to achieve the desired effect using smaller amounts of either factor than would ordinarily be required, thus offering the opportunity to reduce side effects and any toxicity which might be associated with elevated doses.

Therefore, in one aspect, the invention is directed to a pharmaceutical or veterinary composition useful to enhance the function of the immune system of a subject, which composition contains a combination of these two colony stimulating factors.

## Modes for Carrying Out the Invention

### A. The CSF Proteins Useful in the Invention

"Colony stimulating factor-1 (CSF-1)" refers to a protein which exhibits the spectrum of activity understood in the art for CSF-1--i.e., when applied to the standard in vitro colony stimulating assay of Metcalf, D., J Cell Physiol (1970) 76:89, it results in the formation of primarily macrophage colonies. Native CSF-1 is a glycosylated dimer; dimerization may be necessary for activity. Contemplated within the scope of the invention and within the definition of CSF-1 are both the dimeric and monomeric forms. The monomeric form may be converted to the dimer by in vitro provision of intracellular conditions, and the monomer is per se useful as an antigen to produce anti-CSF-1 antibodies.

There appears to be some species specificity: Human CSF-1 is operative both on human and on murine bone marrow cells; murine CSF-1 does not show activity with human cells. Therefore, "human" CSF-1 should be positive in the specific murine radioreceptor assay of Das, S.K., et al, Blood (1981) 58:630, although there is not necessarily a complete correlation. The biological activity of the protein will generally also be inhibited by neutralizing antiserum to human urinary CSF-1 (Das, S.K., et al, supra). However, in certain special circumstances (such as, for example, where a particular antibody preparation recognizes a CSF-1 epitope not essential for biological function, and which epitope is not present in the particular CSF-1 mutein being tested) this criterion may not be met.

Certain other properties of CSF-1 have been recognized more recently, including the ability of this protein to stimulate the secretion of series E prostaglandins, interleukin-1, and interferon from mature macrophages (Moore, R., et al, Science (1984) 223:178). The mechanism for these latter activities is not at present understood, and for purposes of definition herein, the criterion for fulfillment of the definition resides in the ability to stimulate the formation of monocyte/macrophage colonies using bone marrow cells from the appropriate species as starting materials, under most circumstances (see above) this activity may be inhibited by neutralizing antiserum against purified human urinary CSF-1, and, where appropriate for species type, a positive response to the radioreceptor assay. (It is known that the proliferative effect of CSF-1 is restricted to cells of mononuclear phagocyticlineage (Stanley, E.R., The Lymphokines (1981), Stewart, W.E., II, et al, ed, Humana Press, Clifton, NJ), pp. 102-132) and that receptors for CSF-1 are restricted to these cell lines (Byrne, P.V., et al, Cell Biol (1981) 91:848)). Receptors for CSF-1 are known on choriocarcinomama cells related to placental tropoblast (Rettenmier, C.W., et al, J Clin Invest (1986) 77:1740).

The granulocyte colony stimulating factor, G-CSF, is also defined in terms of its activity in certain assays. Human G-CSF causes the differentiation of granulocytes and monocytes from bone marrow progenitor cells. It also induces some leukemia cells to differentiate into more mature cell types; indeed certain myeloid leukemia cells have receptors for G-CSF (Souza, et al, Science (1986) 232:61-65). In general, G-CSF is distinguished from CSF-1 by the nature of the colonies it produces when used to stimulate bone marrow proliferation and in the nature of the antibodies it raises, which are able to interfere with the activity of G-CSF, but not CSF-1, in these assays.

Of course, the two CSF proteins have different amino acid sequences and are basically of different structures, which is reflected in the properties described above. While the proteins usable in the method of the invention are not limited to any specific subspecies, the nature of these proteins can, perhaps, best be understood in terms of the known amino acid sequences of certain representative members of the classes. Representative members of these classes are described below.

The gene encoding a "short form" of CSF-1 produced by MIAPaCa cells is described in Kawasaki et al, Science (1985) supra and incorporated herein by reference. As illustrated in Figure 5 therein, the gene encodes a 224 amino acid monomeric protein. This peptide sequence was deduced from the nucleotide sequence of the cDNA obtained by reverse transcription from enriched messenger prepared from these cells. The sequence matches that encoded by the genomic DNA except for the substitution at position 59 of an aspartic acid residue for the tyrosine shown at that position in the figure. The preparation of DNA encoding these sequences and the preparation of protein by expression of them is disclosed in Kawasaki et al, Science (1985) (supra), and in European Patent Application No. 86901580.0, which corresponds to PCT Publication No. WO86-04607, both of which are incorporated herein by reference.

For convenience, the mature protein amino acid sequence of the monomer shown in Figure 5 of Kawasaki et

3

al, Science (1985) supra, is designated mCSF-1 (mature CSF-1). This protein contains 224 amino acids in the mature sequence and a 32 residue putative signal sequence, which is presumably cleaved upon secretion from mammalian cells. Specifically included in the definition of human CSF-1 are proteins which are mCSF-1, and related forms of mCSF-1 designated by their amino acid differences from mCSF-1 which retain CSF-1 activity, or the subsequently processed forms, thereof, such as glycosylated forms and/or aggregates. Also, CSF-1 derived from other species may fit the definition of "human" CSF-1 by virtue of its display of the requisite pattern of activity as set forth above with regard to human substrate.

For convenience, the amino acid sequence of mCSF-1 will be used as a reference and other sequences which are substantially equivalent to this in terms of CSF-1 activity will be designated by referring to the sequence shown in Figure 5 (the corresponding DNA is encoded in plasmid pcCSF-17). The substitution of a particular amino acid will be noted by reference to the amino acid residue which it replaces. Thus, for example, $ser_{90}CSF-1$ refers to the CSF-1 mutein which has the sequence shown in Figure 5 except that the amino acid at position 90 is serine rather than cysteine. Deletions are noted by an $N\nabla$ followed by the number of amino acids deleted from the N-terminal sequence, or by $C\nabla$ and the number of amino acids remaining followed by a minus sign when residues are deleted from the C-terminal sequence. Thus, $N\nabla_4CSFI$ refers to CSF-1 of Figure 5 wherein the first 4 amino acids from the N-terminus have been deleted; $C\nabla_{130}$- refers to CSF-1 wherein the last 94 amino acids following amino acid 130 have been deleted. Illustrated below are, for example, $asp_{59}CSF-1$ (which contains an aspartic residue encoded by the gene at position 59 rather than the tyrosine residue encoded by the cDNA), $pro_{52}CSF-1$, $glu_{52}CSF-1$, $C\nabla_{150}$-CSF-1, $asp_{59}C\nabla 150$ CSF-1, $asp_{59}C\nabla_{158}$-CSF-1, $\nabla_{158}$-CSF-1, $glu_{52}asp_{59}\nabla_{150}$-CSF-1 and $glu_{52}asp_{59}\nabla_{158}$-CSF-1.

The cloning and expression of any of the CSF-1 and G-CSF muteins used in the present invention can be readily produced following the teachings provided in PCT application publication No. WO 86/04607 using well-known site-directed mutagenesis techniques.

Additional CSF-1 proteins encoded by reverse transcripts of MIAPaCa mRNA contain substantially longer amino acid sequences. These proteins include a 298 amino acid "extra" segment inserted at residue 150 of the pcCSF-17 encoded mCSF-1 protein and expression of this cDNA results in CSF-1 activity in these specific CSF-1 assays, as well. The gene encoding the "long form" of human CSF-1 and the deduced amino acid sequence are described in Ladner et al, EMBO J (1987) supra, and Wong et al, Science (1987) supra, both of which are incorporated herein by reference.

Murine sequences, recovered from L-929 mRNA, a murine fibroblast cell line, are encoded by "long forms" of CSF-1 containing codons for 520 amino acids. These long forms are roughly homologous to the long form of the human protein and apparently contain a 296 amino acid segment corresponding to the "extra" peptide sequence "inserted into" the peptide encoded by pcCSF-17 DNA. Plasmids containing the murine sequences, designated herein pcDBmuCSF53 and pcDBmuCSF-L, respectively, were deposited at the American Type Culture Collection (ATCC) on 7 April 1987 and have CMCC numbers 2892 and 2893, and ATCC numbers 67,248 and 10 67,249, respectively. Modifications similar to those described above may also be made to this longer form.

The abbreviations used herein also include "huCSF-1" for all human forms of the protein and "muCSF1" for murine forms thereof.

It is, of course, well known that bacterially produced mature proteins which are immediately preceded by an ATG start codon may or may not include the N-terminal methionine in the form as produced and recovered. Accordingly, both forms are included. In addition, slight modification of the N-terminal sequence may aid in the processing of the N-terminal methionine; thus deletion of residues 1 and 2 (both glutamic acid) or residues 1 through 3 (glu-glu-val) aids in this manner. Accordingly, these forms are also clearly included in the definition.

The nucleotide and deduced amino acid sequence for human G-CSF is given in Figure 2 of Devlin et al, J Leuk Biol (1987) supra, which is incorporated herein by reference. The gene encodes a 174 amino acid mature protein with a 30 amino acid putative signal sequence.

The G-CSF used in the invention has been cloned and expressed in E. coli using a reverse transcript of mRNA from MIAoaCa cells (see Devlin et al, J Leuk Biol (1987) supra. Muteins involving deletion or substitution of small numbers of amino acids of this primary sequence are also active in assays for G-CSF and are included within the definition. In particular, muteins lacking the N-terminal threonine residue ($N\nabla IG-CSF$), result in protein produced in prokaryotic systems with improved processing of the N-terminal start codon. Also included are muteins wherein the residue at portion 1 is substituted by Ala, Gly, Ala-Ser, or Gly-Gly, and muteins wherein the order of Pro at position 2 and Thr at position 1 is reversed.

Protein with G-CSF activity is also synthesized by LD-1 cells and 5637 cells. The primary structure may not be completely identical to that shown in Figure 2 of Devlin et al, J Leuk Biol (1987) supra. Additional particular muteins of this sequence which are useful are those containing a serine in place of cysteine at position 60 ($ser_{60}$ G-CSF).

All of the colony stimulating factors, and in particular the CSF-1 and G-CSF used in the invention are found natively as dimers and in glycosylated form. The structures shown in the foregoing figures show only the monomeric primary amino acid sequence and these figures are not meant to imply that the polypeptide sequence remains in this form in its biologically active mode. The presence or absence of an N-terminal methionine as a result of bacterial expression also does not destroy activity.

As is the case for all proteins, the precise chemical structure depends on a number of factors. As ionizable amino and carboxyl groups are present in the molecule, a particular protein may be obtained as an acidic or basic salt, or in neutral form. All such preparations which retain their activity when placed in suitable environmental conditions are included in the definition. Further, the primary amino acid sequence may be augmented by derivatization using sugar moieties (glycosylation) or by other supplementary molecules such as lipids, phosphate, acetyl groups and the like, more commonly by conjugation with saccharides. The primary amino acid structure may also aggregate to form complexes, most frequently dimers. Indeed, native human urinary CSF-1 is isolated as a highly glycosylated dimer of 45-90 kd. Certain aspects of such augmentation are accomplished through posttranslational processing systems of the producing host; other such modification may be introduced in vitro. In any event, such modifications are included in the definition so long as the activity of the protein, as defined above, is not destroyed. It is expected, of course, that such modifications may quantitatively or qualitatively affect the activity, either by enhancing or diminishing the activity of the protein in the various assays.

Further, individual amino acid residues in the chain may be modified by oxidation, reduction, or other derivatization, and the protein may be cleaved to obtain fragments which retain activity. Such alterations which do not destroy activity do not remove the protein sequence from the definition.

Modifications to the primary structure itself by deletion, addition, or alteration of the amino acids incorporated into the sequence during translation can be made as described above without destroying the activity of the protein. Such substitutions or other alterations result in proteins having an amino acid sequence which falls within the definition of proteins "having an amino acid sequence substantially equivalent to that of CSF-1 or G-CSF". Also, human and murine derived CSF-1 proteins have nonidentical but similar primary amino acid sequences which display a high homology.

B. Utility and Administration

The CSF-1 and G-CSF proteins of the invention are capable both of stimulating monocyte-precursor/macrophage and granulocyte cell production respectively from progenitor marrow cells, thus enhancing the effectiveness of the immune system, and of stimulating the functions of these differentiated cells such as the secretion of lymphokines in the mature macrophages.

In one application, these proteins are useful as adjuncts to chemotherapy. It is well understood that chemotherapeutic treatment results in suppression of the immune system. Often, although successful in destroying the tumor cells against which they are directed, chemotherapeutic treatments result in the death of the subject due to this side effect of the chemotoxic agents on the cells of the immune system. Administration of G-CSF or CSF-1 to such patients, because of the ability of these factors to mediate and enhance the growth and differentiation of bone marrow-derived precursors into macrophages or granulocytes and to increase the activity of these cells, results in a restimulation of the immune system to prevent this side effect, and thus to prevent the propensity of the patient to succumb to secondary infection. Other patients who would be helped by such treatment include those being treated for leukemia through bone marrow transplants; they are often in an immunosuppressed state to prevent rejection. For these patients also, the immunosuppression could be reversed by administration of G-CSF and CSF-1. G-CSF may be particularly useful to inhibit leukemia growth by causing differentiation to a non-dividing state.

In general, any subject suffering from immunosuppression whether due to chemotherapy, bone marrow transplantation, or other, accidental forms of immunosuppression such as disease (e.g., acquired immune deficiency syndrome) would benefit from the availability of G-CSF and CSF-1 for pharmacological use. In addition, subjects could be supplied enhanced amounts of previously differentiated macrophages or granulocytes to supplement those of the indigenous system, which macrophages or granulocytes are produced by in vitro culture of bone marrow or other suitable preparations treated with CSF-1 and G-CSF. These preparations include those of the patient's own blood monocytes, which can be so cultured and returned for local or systemic therapy.

CSF-1 has been shown to have certain additional specific properties.

The ability of CSF-1 to stimulate production of lymphokines by macrophages and to enhance their ability to kill target cells also makes CSF-1 directly useful in treatment of neoplasms and infections.

CSF-1 stimulates the production of interferons by murine-derived macrophage (Fleit, H.B., et al, J Cell Physiol (1981) 108:347, and human, partially purified, CSF-1 from MIAPaCa cells stimulates the poly IC-induced production of interferon and TNF from human monocytes (Warren, K. and Ralph, P., J Immunol (1986) 137:2281). In addition, CSF-1 stimulates the production of myeloid CSF by human blood monocytes.

Also murine CSF-1 (from L-cell-conditioned medium) stimulates normal C3H/HeN mouse peritoneal macrophages to kill murine sarcoma TU5 targets (Ralph, P. and Nakoinz, I., Cell Immunol (1987) 105:270). This activity is most effective when the CSF-1 is used as pretreatment and during the effector phase. The ability of CSF-1 to do so is much greater than that exhibited by other colony stimulating factors, as shown in Figure 6 hereinbelow. In addition, the ability of murine cells to attack viruses is enhanced by CSF-1 (Lee, M.-T. and Warren, K., J Immunol (1987) 138:3019).

(Murine CSF-1 is inconsistently reported to stimulate murine macrophage to be cytostatic to P815 tumor cells (Wing, E.J., et al, J Clin Invest (1982) 69:270) or not to kill other leukemia targets (Ralph, P, et al, Cell Immunol (1983) 76: 10). Karbassi, A., et al, J Immunol (1987) 139:417, report that CSF-1 may stimulate macrophage to ingest and kill yeast.) Thus, in addition to overcoming immunosuppression per se, CSF-1 can be used to destroy the invading organisms or malignant cells indirectly by stimulation of macrophage secretions and activity.

The lymphokines described herein, G-CSF and CSFI, can be used independently. According to the invention, however, it has now been shown that the effects of one are synergistically enhanced by the presence of the other. Thus, the foregoing activities can be markedly increased by the addition of the two factors substantially simultaneously in effecting treatment. Therefore, formulations containing both of them are particularly useful in the practice of the method of the invention. The dosage levels for the combined therapy are, of course, lower than those desired if either G-CSF or CSF-1 were to be administered alone. Suggested dose ranges for the combination are about 500-50,000 ng/kg for each active ingredient.

The CSF-1 and G-CSF combinations of the invention may be formulated in conventional ways standard in the art for the administration of protein substances. Administration by injection is preferred; formulations include solutions or suspensions, emulsions, or solid composition for reconstitution into injectables. Suitable excipients include, for example, Ringer's solution, Hank's solution, water, saline, glycerol, dextrose solutions, and the like.

In addition, the CSF combinations of the invention may be preincubated with preparations of cells in order to stimulate appropriate responses, and either the entire preparation or the supernatant therefrom introduced into the subject. Materials produced in response to the CSF combination stimulation by various types of blood cells are effective against desired targets, and the properties of these blood cells themselves to attack invading viruses or neoplasms may be enhanced. The subject's own cells may be withdrawn and used in this way, or, for example, monocytes or lymphocytes from another compatible individual employed in the incubation.

It is preferred that the "human" forms of CSF-1 and G-CSF be used in pharmaceutical compositions; however, the murine forms of the protein, CSF-1 protein and G-CSF are particularly useful in convenient model systems in mice to determine the complex pattern of CSF activities.

The method of the invention involves administering to a warm-blooded animal host, including mouse, rabbit, primate, avian, or other warm-blooded species a pharmacologically effective amount (in combination) of G-CSF and CSF-1. The two lymphokines may be combined in vitro before administration, as neither is adversely affected chemically and both remain efficacious. They may, however, be separately administered to the patient, either in order or simultaneously, but in any event, as part of the same treatment protocol.

Administration may take place by any suitable technique including parenteral administration, such as intravenous, interarterial, intramuscular, subcutaneous or combinations of the above. The subject may be treated locally for a particular tumor, for example, or systemically. The dose and dosage regimen depends on the nature of the malady, the particular formulation decided upon, and the mode of administration.

Administration is in an amount which is non-toxic to the host. Toxicity may be monitored by the extent and type of various side affects such as fever, chills and general malaise.

## C. Preparation of the CSF Factors

The proteins useful as active ingredients in the compositions of the invention are prepared either by direct isolation from the cells producing them, or by recombinant techniques. Preparation of these proteins using recombinant means is extensively described in EPO Application Publication No. 86901580.0 cited above.

The MIAPaCa-2 cell line, described by Yunis, A.A. et al, Experimental Hematol (1984) 12:838-843, is available from the American Type Culture Collection, 12301 Parklawn Avenue, Bethesda, Maryland 20895 under Accession No. ATCC CRL1420.

The LD-1 cell line, described by Lilly, M.B. and Rado, T.A. (Blood (1984) 64..130a), was also deposited in assignee's culture collection prior to 31 December 1986.

Sources for equivalent initial G-CSF are disclosed in Devlin, et al, J Leukocyte Biol (1987) supra.

In one illustration below, CSF-1 was obtained from the supernatant of stimulated MIAPaCa cells and G-CSF was prepared from the supernatant of LD-1 cells. In another illustration, the G-CSF and CSF-1 of the invention were prepared recombinantly as referenced above. Either approach is suitable.

MIAPaCa CSF-1 was produced serum-free by induction with phorbol myristic acetate, and the CSF-1 is purified from the cell supernatant using the procedure of Csejtey, J. and Boosman, A., Biochem Biophvs Res Comm (1986) 138:238. The supernatant is subjected to calcium phosphate gel chromatography (according to Das), followed by affinity chromatography using lentil lectin (in place of the ConA affinity step of Das), and then to an immunoaffinity step employing the YYG106 monoclonal antibody conjugated to Sepharose B.

The details of this method is similar to those employed to purify human urinary CSF-1. The urinary protein is partially purified by standard methods as described by Das, S. K., et al, Blood (1981) 58:630, followed by an affinity purification step using a rat monoclonal antibody to murine CSF-1, designated YYG106, attached to a Sepharose B column (Stanley, E.R., Methods Enzymol (1985) 116:564).

G-CSF was purified from serum-free LD-1 supernatant by phenyl Sepharose CL-4B chromatography using a procedure similar to that of Nicola, N.A. et al, Nature (1985) 314:625. This was followed by size exclusion on S200 and by reverse phase HPLC as described by Welte, et al Proc Natl Acad Sci (1985) 82:1526. Elution is with i-propane or acetonitrile.

## Example: The Bone Marrow Proliferation Assay

The synergistic effect of G-CSF and CSF-1 was measured using an assay for the formation of colonies from bone marrow.

In the assay, bone marrow cells from Balb/C mice are treated with serial dilutions of the test substance.

Proliferation of the cells can be measured by uptake of labeled thymidine, essentially as described by Moore, R.N., et al, J Immunol (1983) 131:2374; Prystowsky, M.B., et al, Am J Pathol (1984) 114:149, however in the results below, formation of colonies is assessed using the method of Stanley, E.R., et al J Lab Clin Med (1972) 79:657). In this method, 1 unit of colony stimulating activity is defined as the amount of CSF needed to produce one colony from $10^5$ bone marrow cells/ml.

More particularly, bone marrow cells isolated from Balb/C mice were subjected to Ficoll-Hypaque centrifugation to separate the mononuclear cells. These mononuclear cells were then cultured in 35 mm wells containing $10^5$ cells per ml of 0.3% agar with 10% fetal calf serum and either CSF-1 or G-CSF or both. The wells were incubated for 7 days at 37°C under 5% $CO_2$ and the number of colonies (groups of more than 40 cells) in each well was counted. Some colonies were removed from the dishes, cytocentrifuged and stained to determine colony morphology.

The results at two levels of G-CSF and CSF-1 alone and in combination are shown in Table 1. In this example the CSF-1 was isolated from the supernatant of MIAPaCa cells and the G-CSF from LD-1 cells as described above.

## Table 1

### COLONIES

| G-CSF | CSF-1 | G-CSF + CSF-1 |
|---|---|---|
| 20 (about 20 units) | 14 (about 14 units) | 48 (same conc.) |
| 5 (about 10 units) | 1 (about 7 units) | 17 (same conc.) |

COLONY TYPE resulting from first assay:

| | | |
|---|---|---|
| 0% M | 100% M | 25% M |
| 33% MN | 0% MN | 75% MN |
| 67% N | 0% N | 0% N |

(M = macrophage, N = neutrophil, MN represents a mixed colony)

The synergistic effect is evident. In assessing this activity, the preferred levels of the components are those which represent below approximately half-maximum value for the individual factors. I.e., for each factor, activity increases with increasing amount to a plateau; the useful levels for the assay are below those at the middle of the steep portion of the curve.

Table 2 shows the results of a similar assay using duplicate assays confirming the results in Table 1, but wherein recombinantly produced proteins were used. The G-CSF was produced in E. coli as described in U.S. Serial No. 932,037 (supra); the CSF-1 was produced in CV-1 cells as described in EPO Application No. 86901580.0 (supra).

## Table 2

| G-CSF (25 units) | CSF-1 (30 units) | G-CSF + CSF-1 (25 units) (30 units) |
|---|---|---|
| 25 | 31 | 117 |
| 23 | 31 | 128 |

As shown in these experiments, the effect of G-CSF and CSF-1 is synergistic. The combined effect of these factors is greater, often several fold, than the sum of the effects independently. This is particularly true at low concentration. The character of the colonies formed is also altered. As expected, CSF-1 alone results in the

**0 273 778**

formation exclusively of macrophage colonies, whereas solely macrophage colonies are not formed using G-CSF. G-CSF alone produces colonies containing neutrophils or macrophage + neutrophils. However, when the combination is used, the majority of the colonies are macrophage and neutrophils, with a minority of macrophage colonies.

Similar preliminary experiments using human bone marrow cells showed a moderate synergy as illustrated in Table 3 below.

In this assay, human bone marrow cells were treated with suitable dilutions of each test substance alone and in combination according to the procedure described by Broxmeyer, H.E., et al, J Immunol (1986) 136:4487-4495. In this example, both CSF-1 and G-CSF were isolated from the supernatant of MIAPaCa cells and LD-1 cells, respectively.

## Table 3

| Samples[a] | Colonies at Day 7 |
|---|---|
| McCoy's Medium (control) | 0 |
| G-CSF (6 units) | 6 ± 2 |
| CSF-1 (500 units) | 18 ± 3 |
| G-CSF (6 units) and CSF-1 (500 units) | 36 ± 3 |

[a]Samples were plated at 10% v/v with $1 \times 10^5$ non-adherent low density normal human bone marrow cells/ml.

Example: In Vivo Activity of CSF-1 and G-CSF

The synergistic effect of G-CSF and CSF-1 was measured using BDF₁ mice according to the procedure described in Broxmeyer, H.E., et al, Proc Natl Acad Sci USA (1987) 84:3871-3875.

Briefly, BDF₁ mice were pretreated with 100 ug of purified, endotoxin-depleted, iron-saturated human lactoferrin i.v. Pretreatment with lactoferrin places the progenitor cells into a slow or noncycling state. Three hours later, the mice were treated i.v. with sterile pyrogen-free saline or various concentrations of purified endotoxin-free recombinant human G-CSF (rhu G-CSF) or recombinant human CSF-1 (rhu CSF-1), alone or in combination. Mice were sacrificed 24 hours after the administration of CSF and marrow and spleen cells from these mice were assessed in vitro for the proliferation of CFU-GM (percentage of cells in DNA synthesis).

### Table 4

| Test Material Given In Vivo | % CFU-GM in S-Phase | |
| --- | --- | --- |
| | Bone Marrow | Spleen |
| Saline (Control Diluent) | 11 ± 3 | 6 ± 3 |
| rhu G-CSF (10,000 units) | 47 ± 5[a] | 43 ± 4[a] |
| rhu G-CSF (2,000 units) | 15 ± 3 | 7 ± 4 |
| rhu CSF-1 (10,000 units) | 50 ± 4[a] | 30 ± 5[a] |
| rhu CSF-1 (2,000 units) | 13 ± 5 | 9 ± 4 |
| rhu G-CSF (50 units) + rhu CSF-1 (50 units) | 44 ± 3[a] | 29 ± 5[a] |

[a] $p$ at least $< 0.005$.

As seen in Table 4, relatively high concentrations of each molecule alone increased the cycling rates of CFU-GM, and when low concentrations of G-CSF and CSF-1, which alone were inactive, were administered simultaneously to the mice, the CFU-GM were placed into rapid cell cycle.

**Claims**

1. Use of the combination of G-CSF and CSF-1 for the manufacture of a composition useful to effect syneristic enhancement of the immune system of a subject.

2. A composition effecting synergistic enhancement of the immune system of a subject comprising G-CSF and CSF-1.

3. A composition of claim 2 wherein the G-CSF is human G-CSF.

4. A composition of claim 3 wherein the human G-CSF has the amino acid sequence corresponding to that sequence obtained from MIAPaCa cells or an allelic variant thereof.

5. A composition of claim 4 wherein the human G-CSF is an N ∇ - and/or C ∇ -mutein.

6. A composition of any one of claims 2 to 5 wherein the CSF-1 is human CSF-1.

7. A composition of claim 6 wherein the human CSF-1 has the 224 amino acid sequence corresponding to that sequence obtained from M]APaCa cells or an allelic variant thereof.

8. A composition of claim 6 wherein the human CSF-1 has the 522 amino acid sequence corresponding to that sequence obtained from MIAPaCa cells or an allelic variant thereof.

9. A compostion of claims 7 or 8 wherein the human CSF-1 is an N ∇ - and/or C ∇ -mutein.

10. The use of G-CSF of CSF-1 in preparing a medicament or medication for simultaneous or separate delivery of the G-CSF and CSF-1 to an animal whereby a syneristic biological effect is achieved.